(19) Europäisches Patentamt
European Patent Office
Office européen des brevets

(11) Publication number: 0 308 573 A2

# (12) EUROPEAN PATENT APPLICATION

(21) Application number: 88104107.3

(22) Date of filing: 10.04.84

(51) Int. Cl.[4]: C07D 233/84 , A61K 31/415

(30) Priority: 12.04.83 US 484122
19.03.84 US 590665

(43) Date of publication of application:
29.03.89 Bulletin 89/13

(60) Publication number of the earlier application in accordance with Art.76 EPC: 0 125 033

(84) Designated Contracting States:
AT BE CH DE FR GB IT LI LU NL SE

(71) Applicant: SMITHKLINE BECKMAN CORPORATION
One Franklin Plaza P O Box 7929
Philadelphia Pennsylvania 19103(US)

(72) Inventor: Frazee, James Simpson
310B Sutton Towers
Collingwood New Jersey 08108(US)
Inventor: Kaiser, Carl
1105 Sylvan Drive
Haddon Heights New Jersey 08035(US)
Inventor: Kruse, Lawrence Ivan
646 Clinton Avenue
Haddonfield New Jersey 08033(US)

(74) Representative: Giddings, Peter John, Dr. et al
Smith Kline & French Laboratories Ltd.
Corporate Patents Mundells
Welwyn Garden City Hertfordshire AL7 1EY(GB)

## (54) Intermediates useful in the preparation of dopamine-beta-hydroxylase inhibitors.

(57) Compounds of Structure (III) :

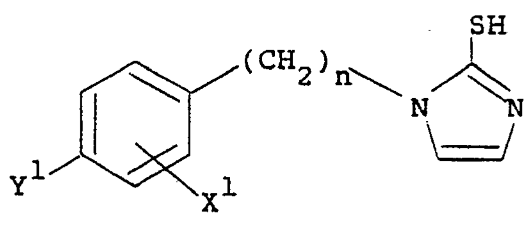

(III)

in which $Y^1$ is $-OCH_3$; $X^1$ is -H, halogen, $C_{1-4}$ alkyl, -CN, $-NO_2$, $-SO_2NH_2$, $-CO_2H$, $-CONH_2$, -CHO, $-CH_2OH$, $-CF_3$, $-OCH_3$, $-SO_2C_{1-4}$ alkyl, $-SO_2C_{1-4}$ fluoralkyl, or $-CO_2C_{1-4}$ alkyl; and useful as intermediates in the preparation of novel dopamine $\beta$-hydroxylase inhibitors.

EP 0 308 573 A2

# Intermediates useful in the preparation of Dopamine-β-hydroxylase Inhibitors

This application is a divisional of European Patent Application No. 84302423.3 (Publication No. 0125033).

The present invention relates to intermediates useful in the preparation of inhibitors of dopamine-β-hydroxylase.

In the catecholamine biosynthetic pathway, tyrosine is converted in three steps to norepinephrine (NE). Intermediates are dihydroxyphenylalanine (DOPA) and dopamine (DA). The latter is hydroxylated to norepinephrine by dopamine-β-hydroxylase (DBH) in the presence of oxygen and ascorbic acid.

Inhibition of catecholamine activity has been found to decrease hypertension. See, for example, Matta et al., Clin. Pharm. Ther. 14, 541 (1973), and Teresawa et al., Japan Circ. J. 35, 339 (1971). Weinshilboum, Mayo Clin. Proc. 55, 39 (1980), reviews compounds which inhibit catecholamine activity by interfering with adrenergic receptors. Alternatively, the catecholamine biosynthetic pathway can be suppressed at any of the three steps, resulting in decreased levels of NE. In addition to decreasing hypertension, inhibitors of NE synthesis are active as diuretics, natriuretics, cardiotonics and vasodilators. Inhibition of DBH activity can have the added advantage of increasing levels of DA, which as reported by Ehrreich et al., "New Antihypertensive Drugs," Spectrum Publishing, 1976, pp. 409-432, has been found to have selective vasodilator activity at certain concentrations.

DBH inhibitors have also been shown to reduce or prevent formation of gastric ulcers in rats by Hidaka et al., "Catecholamine and Stress," edit. by Usdin et al, Permagon Press, Oxford, 1976, pp. 159-165 and by Osumi et al., Japan. J. Pharmacol. 23, 904 (1973).

Although there are many known inhibitors of DBH, none of these agents has found clinical application because of non-specific, often toxic, properties they possess. Fusaric acid, for example, has been found to be hepatotoxic. See, for example, Teresawa et al., Japan. Cir. J. 35, 339 (1971) and references cited therein. Presumably, the picolinic acid structure interacts with a number of metalloproteins and enzymes in non-specific fashion to produce observed side effects.

In UK specification 1,155,580 are disclosed compounds having the formula:

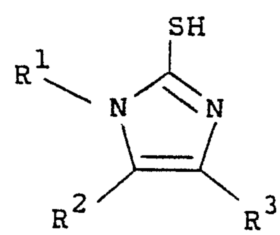

wherein $R^2$ and $R^3$ can be H and $R^1$ can be substituted phenyl. The compounds are said to have analgesic, antiinflammatory and antipyretic properties. Gebert et al., U.S. Patent 3,915,980, disclose such compounds wherein $R^1$ can be phenyl or phen($C_{1-3}$) alkyl, as intermediates to imidazolyl-2-thioalkanoic acid esters.

Iverson, Acta Chem. Scand. 21, 279 (1967) reports a compound having the formula;

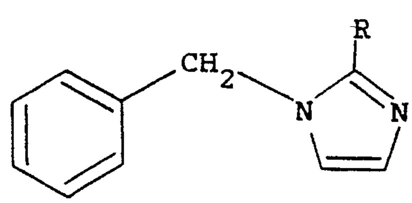

wherein R can be $-CO_2H$ or $-CH_2NHC_6H_5$, but does not report a pharmaceutical use for the compound.

European Patent Application No. 84302423.3 discloses inhibitors of DBH having a mercaptoimidazole moiety and a phenethylamine analogue moiety. More particularly, the application discloses selected novel compounds having the formula:

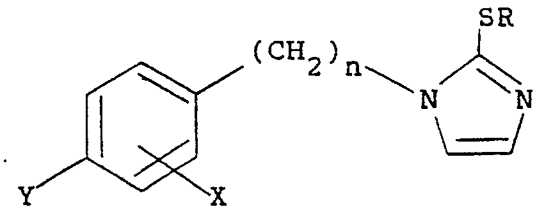

(I)

wherein
X is -H, -OH, halogen, $C_{1-4}$ alkyl, -CN,-$NO_2$,-$SO_2NH_2$, -$CO_2H$, -$CONH_2$, -CHO, -$CH_2OH$, -$CF_3$, -$OCH_3$, -$SO_2C_{1-4}$ alkyl, -$SO_2C_{1-4}$ fluoroalkyl, -$CO_2C_{1-4}$ alkyl or any synthetically accessible combination thereof up to four substituents;
Y is -H, -OH, halogen, $C_{1-4}$ alkyl, -CN, -$NO_2$,-$SO_2NH_2$, -$CO_2H$, -$CONH_2$, -CHO, -$CH_2OH$, -$CF_3$, -$SO_2C_{1-4}$ alkyl, -$SO_2C_{1-4}$ fluoroalkyl, or -$CO_2C_{1-4}$ alkyl; R is -H or $C_{1-4}$ alkyl; and
n is 0-4,
or a hydrate or, when R is $C_{1-4}$ alkyl, a pharmaceutically acceptable acid addition salt thereof, provided that when n is 0, Y is -OH and when n is 1-3, at least one of Y and X is not -H.

In preferred compounds of the application, Y is -OH; R is -H; n is 1 or 3; and X is -H, -OH or halogen (in particular, 3,5-dichloro, 3,5-difluoro, 3-chloro, or 3-fluoro) or Y is -H; R is -H; n is 1 or 3 and X is halogen (in particular, 3,5-dichloro, 3,5-difluoro, 3-chloro, or 3-fluoro). In the most preferred compound, Y is -H, X is 3,5-difluoro, R is -H and n is 1.

It is intended that the above formulae include the tautomer of the compounds wherein R is -H, that is, the compounds having the above formulae wherein the imidazole moiety has the formula:

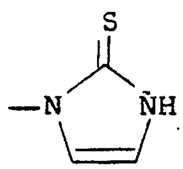

The above formulae also include hydrates of the compounds and pharmaceutically acceptable acid addition salts of the compounds wherein R is $C_{1-4}$ alkyl. The invention also includes pharmaceutical compositions comprising the compounds having the above formulae, provided that when n is 0, Y is -OH, and pharmaceutical carriers.

The present invention relates to intermediates for the preparation of compounds (I), in which Y is OH and R is hydrogen, said intermediates having the formula:

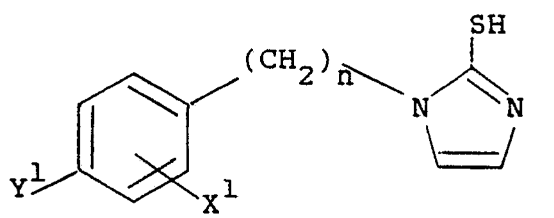

(III)

wherein $X^1$ is the same as X in formula (I), n is 0 to 4 and $Y^1$ is $OCH_3$.

The compounds of EPA 84302423.3 can be prepared from corresponding starting benzyl or phenyl compounds such as benzaldehydes, which are known and described in published references or are readily accessible, by known techniques such as illustrated in Scheme I, below, wherein $X^1$ and $Y^1$ are X and Y, respectively, except that when Y is -OH, $Y^1$ is -$OCH_3$ and when X is -OH, $X^1$ is -$OCH_3$. As illustrated, n is one, although n can be 0-4. Scheme I illustrates reductive amination of benzaldehydes (I) with an aminoacetaldehyde acetal followed by reduction by, for example, catalytic hydrogenation or treatment with a reducing agent such as $NaBH_4$, $LiAlH_4$ or $AlH_3$, to provide intermediate substituted benzylamines (II). Upon reaction with acidic thiocyanate, the intermediates (II) yield mercaptoimidazole products (III). The mercaptoimidazole products can be prepared from other than benzaldehydes, as illustrated in Examples 1 and 4, below.

Scheme I

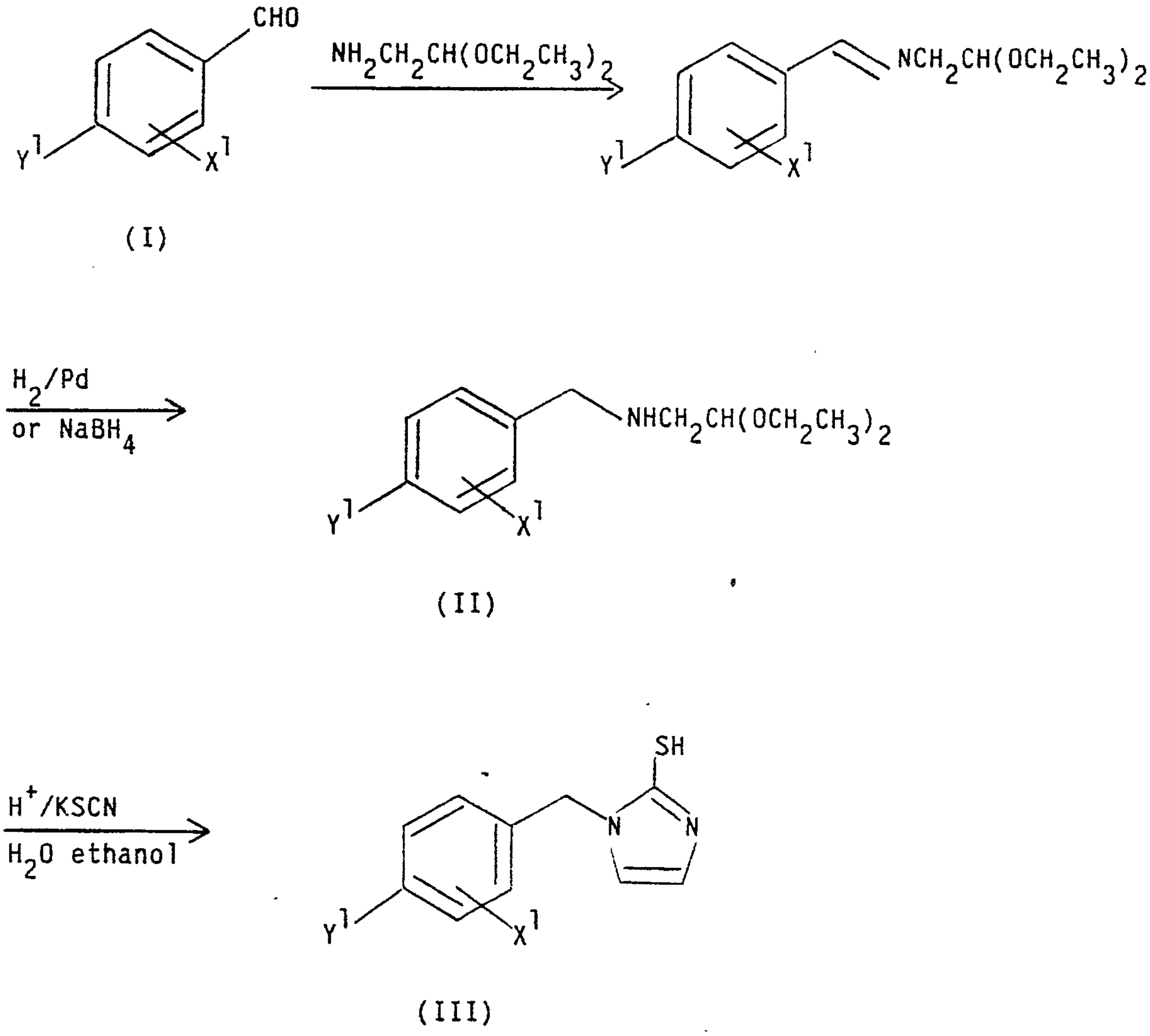


(I)

(II)

(III)

The 1-phenyl substituted 2-mercaptoimidazoles (n is 0) are preferably prepared by reaction of an appropriately substituted phenyl isothiocyanate with an aminoacetaldehyde acetal followed by strong acid catalyzed cyclization, as illustrated in Example 1, below.

The compounds wherein n is 2,3 or 4 are preferably prepared as illustrated in Example 4 and in Examples 23 and 24, below. Coupling of substituted phenylalkanoic acids as the acid halides, preferably chlorides, with aminoacetaldehyde acetals and subsequent reduction provides such intermediate substituted phenyl alkylamines.

$Y^1$ in Scheme I is the same as Y except that when Y is -OH, $Y^1$ is $-OCH_3$, deprotection of the 4-alkoxy group with, for example, $BBr_3$ or HBr, or nucleophilic aromatic substitution with dilute hydroxide, provides the phenol (Y is -OH). $X^1$ may be one or more substituents at the 2-, 3-, 5- or 6- positions, provided the combination of substituents is accessible, that is, does not result in significant instability due to steric hindrance. When $X^1$ is $-OCH_3$, it can be deprotected as described above for $Y^1$.

The compounds of EPA 84302423.3 in which R is $C_{1-4}$ alkyl are preferably prepared by allowing the deprotection with for example, $BBr_3$, in an alkanol to proceed to formation of an alkyl bromide which alkylates the mercapto group as illustrated in Example 6, below. Alternatively, a solution or suspension of an appropriately substituted mercaptoimidazole in an inert solvent, for example, methanol, tetrahydrofuran and aqueous dimethylformamide, can be reacted with an alkylating agent, for example, alkyl iodide, bromide or tosylate. Methyl iodide is preferred in this alternative procedure.

The pharmaceutically acceptable acid addition salts of the compounds wherein R is $C_{1-4}$ alkyl are formed with strong or moderately strong organic or inorganic acids by methods known to the art. For

example, the base is reacted with an inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Exemplary of the salts which are included in this invention are maleate, fumarate, lactate, oxalate, methanesulfonate, ethanesulfonate, benzenesulfonate, tartrate, citrate, hydrochloride, hydrobromide, sulfate, phosphate and nitrate salts.

The compounds of EPA 84302423.3, because they can be used to inhibit DBH activity, have therapeutic value as diuretic, natriuretic, cardiotonic, antihypertensive and vasodilator agents, as well as antiulcerogenic and antiparkinson disease agents.

The following examples are illustrative of utility of the compounds of the invention as intermediates in the preparation of the dopamine β-hydroxylase inhibitors of EPA 84302423.3. The starting compounds are commercially available or are prepared by known techniques. All temperatures and melting points (m.p.) are in degrees Celsius (° C).

## Example 1

A solution of 10 g (.06 mole) of p-methoxyphenylisothiocyanate in 100 ml of $CHCl_3$ was treated with 6.3 g (.06 mole) of aminoacetaldehyde dimethyl acetal. The solvent was evaporated and the residue was recrystallized from ethanol to yield N-(p-methoxyphenyl)-N′-(β,β-dimethoxyethyl)thiourea, 9.2 g (57%). A suspension of this thiourea in a solution of 5 ml of concentrated $H_2SO_4$ and 20 ml of $H_2O$ was refluxed for 3 hours. The mixture was cooled and a solid was filtered, washed with $H_2O$ and dried. Recrystallization from ethanol gave 1-(4-methoxyphenyl)-2-mercaptomidazole, 4.9 g (70%), m.p. 215-7°. The compound is deprotected, for example, as illustrated in Example 5 and 6, below to prepare the phenol, Y is -OH.

## Example 2

A mixture of 13.6 g (0.1 mole) of anisaldehyde, 13.3 g (0.1 mole) of aminoacetaldehyde diethyl acetal and 1 ml of $CH_3OH$ was heated at 95° for 10 minutes. A residue was dissolved in 150 ml of ethanol and hydrogenated over 10% Pd on carbon at 50 psi (0.34 MPa) until $H_2$ uptake was complete. The catalyst was filtered and the filtrate was treated with 10.4 g (0.107 mole) of KSCN, 40 ml of 3N HCl and 40 ml of $H_2O$. The mixture was refluxed, letting the solvent evaporate until the volume of the reaction mixture was 100 ml. After 45 minutes, the mixture was cooled, and a solid was filtered, washed with $H_2O$ and dried. Recrystallization from ethanol gave (1-(4-methoxybenzyl)-2-mercaptomidazole, 15.0 g (68%), m.p. 140-142°.

## Example 3

A solution of 10.75 g (.05 mole) of 3-bromo-4-methoxybenzaldehyde and 6.65 g (.05 mole) of aminoacetaldehyde diethyl acetal in 25 ml of ethanol was refluxed for 30 minutes. The solvent was evaporated and the residue was dissolved in $CH_2Cl_2$. The $CH_2Cl_2$ solution of the Schiff base was washed with saturated aqueous NaCl, dried ($K_2CO_3$)and filtered, and the solvent was evaporated. Residual Schiff base was dissolved in 100 ml of methanol, cooled to 5°, and treated with 5.0 g of $NaBH_4$. The reaction mixture was allowed to warm to 22° and, after 4 hours, the solvent was evaporated. The residue was taken up in diethyl ether, washed with $H_2O$, dried ($MgSO_4$) and filtered, and the solvent was evaporated. A solution of the residue in $CHCl_3$, upon treatment with ethereal HCl gave, on standing, crystals of N-(3-bromo-4-methoxybenzyl) aminoacetaldehyde diethylacetal hydrochloride, 10.75 g (58%), m.p. 112-120°.

A solution of 10.74 g (.029 mole) of N-(3-bromo-4-methoxybenzyl)aminoacetaldehyde diethyl acetal hydrochloride and 3.37 g (0.35 mole) of KSCN in 50 ml of $H_2O$, 50 ml of ethanol and 5 ml of 3N HCl was refluxed for 4.5 hours. One hundred ml of $H_2O$ was added and the mixture was cooled. A solid was filtered, washed with $H_2O$ and dried. Recrystallization from ethanol gave 1-(3-bromo-4-methoxybenzyl)-2-mercaptoimidazole, 6.3 g (72%). m.p. 188°.

Example 4

A solution of 12.5 g (.07 mole) of p-methoxyphenylpropionic acid in 100 ml of $CH_2Cl_2$ and one drop of pyridine was treated with 9.8 g (.077 mole) of oxalyl chloride. After 2.5 hours, the solvents were thoroughly evaporated to give the acid chloride as an oil. A solution of the acid chloride in 100 ml of $CH_2Cl_2$ was slowly added to a cold (0°) solution of 14.7 g (0.14 mole) of aminoacetaldehyde dimethyl acetal in 300 ml of $CH_2Cl_2$ at a rate such that the temperature stayed below 20°. After 1 hour, the reaction mixture was poured into $H_2O$, and the $CH_2Cl_2$ layer was separated and washed with aqueous $Na_2CO_3$, 0.5N HCl and $H_2O$. Following drying and evaporation of the solvent, N-($\beta$,$\beta$-dimethoxyethyl)-p-methoxyphenyl-propionamide was left as a solid, 10.3 g (55%). A solution of this amide in 300 ml of diethyl ether was slowly added to a slurry of 4.0 g of $LiAlH_4$ in 400 ml of diethyl ether and 350 ml of tetrahydrofuran (THF). After 3.5 hours at 22°, excess $LiAlH_4$ was cautiously destroyed, the reaction mixture was filtered and the filtrate was evaporated. The residue was dissolved in 100 ml of 0.15N HCl, washed with diethyl ether, basified with $NaHCO_3$ and extracted with diethyl ether. The extracts were dried ($MgSO_4$)and the solvent was evaporated to give N-[3-(4-methoxyphenyl)propyl]aminoacetaldehyde dimethyl acetal, 4.6 g (52%), as an unstable oil.

A solution of 3.62 g (.014 mole) of N-[3-(4-methoxyphenyl)propyl]aminoacetaldehyde dimethyl acetal and 1.4 g (.0144 mole) of KSCN in 20 ml of ethanol, 5 ml of $H_2O$ and 2 ml of concentrated HCl was refluxed for five hours. Fifty ml of $H_2O$ was added, the mixture was cooled and a solid was filtered, washed with $H_2O$ and dried. Recrystallization from ethanol gave 1-[3-(4-methoxyphenyl)propyl]-2-mercaptoimidazole, 2.4 g (69%), m.p. 108-109°.

Example 5

A solution of 1.75 g (.007 mole) of 1-[3-(4-methoxyphenyl)propyl]-2-mercaptoimidazole in 60 ml of $CH_2Cl_2$ was deprotected by treatment with a solution of 7.0 g (.028 mole) of $BBr_3$ in 10 ml of $CH_2Cl_2$. After 1.5 hours, the reaction mixture was cooled to 0°, and methanol was cautiously added. After a vigorous reaction subsided, the solvents were evaporated. The residue was recrystallized from ethanol to give 1-[3-(4-hydroxyphenyl)propyl]-2-mercaptoimidazole, 1.02 g (67%), m.p. 185°.

Example 6

A solution of 1.2 g (.0046 mole) of 1-[3-(4-methoxyphenyl)propyl]-2-mercaptoimidazole in 40 ml of $CH_2Cl_2$ was treated with a solution of 3.5 g (.014 mole) of $BBr_3$ in 10 ml of $CH_2Cl_2$. After 4 hours, methanol was cautiously added, the mixture was stirred for an additional 18 hours, and the solvents were evaporated. The residue was dissolved in $H_2O$, washed with ethyl acetate, neutralized with $NaHCO_3$ and extracted with ethyl acetate. The extracts were dried ($MgSO_4$)and filtered and the solvent was evaporated. The residue was dissolved in 5 ml of ethanol, and treated with ethereal HCl. A crystalline product was filtered and recrystallized from ethanol, to give 1-[3-(4-hydroxy- phenyl)propyl]-2-thiomethylimidazole hydrochloride, 0.61 g (45%), m.p. 140-142°.

Example 7

A solution of 1-[3'-nitro-4'-methoxybenzyl]-2-mercaptoimidazole (1.59 g, 6.0 moles) in 10% aqueous NaOH (200 ml) was refluxed for two hours, cooled, acidified with concentrated HCl, cooled and filtered. The crystalline product was washed with water. Recrystallization from ethanol provided 1.25 g (80%) of product as yellow prisms, m.p. 225-227° (dec).

Example 8

A mixture of 1-[2',6'-dichloro-4'-methoxybenzyl]-2-mercaptoimidazole (1 g) in concentrated aqueous hydrobromic acid (50 ml) was heated at reflux under argon for 1.25 hours, and then cooled. The product was collected by filtration. Washing with concentrated aqueous hydrobromic acid and water and drying yielded 0.64 g (60%) of product as light yellow crystals: m.p. 235° (dec).

Example 9

A mixture of 1-[3'-trifluoromethyl-4'-methoxybenzyl]-2-mercaptoimidazole (2.0 g) and pyridine hydrochloride (15 g) was melted at 210° for 30 minutes, cooled, diluted with water and extracted with ethyl acetate. The ethyl acetate extracts were treated with charcoal, dried over sodium sulfate and concentrated to give a thick oil. Addition of THF (3 ml), ether (6 ml), and hexane (15 ml) produced yellow crystals. Recrystalization from ethyl acetate/ hexane yielded 0.7 g (37%) of cream colored crystals: m.p. 220° (dec).

Example 10

1-[3',5'-Dichlorobenzyl]-2-mercaptoimidazole

A mixture of 3,5-dichlorobenzaldehyde (17.5 g, 0.1 mole) and aminoacetaldehyde diethylacetale (13.3 g, 0.1 mole) was heated on a steam bath. The resulting solution was stirred at room temperature during slow addition of sodium borohydride (3 g, 0.08 mole) and the mixture was stirred overnight at room temperature. The mixture was concentrated under reduced pressure, and the residue was partitioned between ethyl acetate and water. The ethyl acetate layer was washed sequentially with water and brine, and then dried over anhydrous sodium sulfate, and concentrated under reduced pressure. The resulting oil was heated at reflux with water (100 ml), concentrated hydrochloric acid (22 ml), ethanol (41 ml), and potassium thiocyanate (10.7 g, 0.11 mole) for 2 hours. The mixture was cooled and diluted with water (250 ml), and the crude product was collected by filtration and dried. Recrystallization twice from acetic acid provided 7.3 g (30%) of the title compound as light yellow crystals: m.p. 209-211°.

Example 11

1-[2',6'-Dichlorobenzyl]-2-mercaptoimidazole

Reaction of 2,6-dichlorobenzaldehyde (17.5 g, 0.1 mole) and aminoacetaldehyde diethylacetal (13.3 g, 0.1 mole) substantially as above yielded 5.4 g (21%) of the title compound as white needles: m.p. 242-3° (ethanol/ether).

Example 12

1-[2'-Chlorobenzyl]-2-mercaptoimidazole

Reaction of 2-chlorobenzaldehyde (14 g, 0.1 mole) and aminoacetaldehyde diethylacetal (13.3 g, 0.1 mole) substantially as above yielded 11.2 g (50%) of the title compound as white crystals: m.p. 206-7° (acetone/ethanol).

Example 13

1-[2',5'-Dichlorobenzyl]-2-mercaptoimidazole

Reaction of 2,5-dichlorobenzaldehyde (10.25 g, 0.059 mole) and aminoacetaldehyde diethylacetal (7.79 g, 0.059 mole) substantially as above yielded 5.2 g (34%) of the title compound as white crystals: m.p. 265° (dec) (propionic acid).

Example 14

1-[4'-Chlorobenzyl]-2-mercaptoimidazole

Reaction of 4-chlorobenzaldehyde (14 g, 0.1 mole) and aminoacetaldehyde diethylacetal (13.3 g, 0.1 mole) substantially as above yielded 8.3 g (36%) of the title compound as white crystals: m.p. 187-9° (acetonitrile).

Example 15

1-[2',3'-Dichlorobenzyl]-2-mercaptoimidazole

Reaction of 2,3-dichlorobenzaldehyde (8.7 g 0.05 mole) and aminoacetaldehyde diethylacetal (6.65 g, 0.05 mole) substantially as above yielded 3.0 g (23%) of the title compound as white crystals: m.p. 195-7° (ethanol).

Example 16

1-[4'-Fluorobenzyl]-2-mercaptoimidazole

Reaction of 4-fluorobenzaldehyde (12.4 g, 0.1 mole) and aminoacetaldehyde diethylacetal (13.3 g, 0.1 mole) substantially as above yielded 13.0 g (62.5%) of the title compound as white crystals: m.p. 167-9° (ethanol).

Example 17

1-[3',4'-Dichlorobenzyl]-2-mercaptoimidazole

Reaction of 3,4-dichlorobenzaldehyde (17.5 g, 0.1 mole) and aminoacetaldehyde diethylacetal (13.3 g, 0.1 mole) substantially as above yielded 10 g (39%) of of the title compound as white crystals: m.p. 178-81° (ethanol).

Example 18

1-[2',4'-Dichlorobenzyl]-2-meraptoimidazole

Reaction of 2,4-dichlorobenzaldehyde (17.5 g, 0.1 mole) and aminoacetaldehyde diethylacetal (13.3 g, 0.1 mole) substantially as above yielded 8.5 g (33%) of the title compound as of white crystals: m.p. 185-7° (2-propanol).

Example 19

1-[3'-Chlorobenzyl]-2-mercaptoimidazole

Reaction of 3-chlorobenzaldehyde (14 g, 0.1 mole) and aminoacetaldehyde deithylacetal (13.3 g, 0.1 mole) substantially as above yielded 14 g (62.5%) of the title compound as white crystals: m.p. 129-131° (acetonitrile).

Example 20

1-[2′,4′,6′-Trichlorobenzyl]-2-mercaptoimidazole

Reaction of 2,4,6-trichlorobenzaldehyde (20.9 g, 0.1 mole) and aminoacetaldehyde diethylacetal (13.3 g, 0.1 mole) substantially as above yielded 12 g (46%) of the title compound as white crystals: m.p. 240-4° (ethanol).

Example 21

1-[3-Fluorobenzyl]-2-mercaptoimidazole

Reaction of 3-fluorobenzaldehyde (24.8 g, 0.2 mole) and aminoacetaldehyde diethyl acetal (26.6 g, 0.2 mole) substantially as above yielded 28 g (67%) of the title compound as white crystals: m.p. 112.5-114° (2-propanol/water).

Example 22

1-[3′,5′-Difluorobenzyl]-2-mercaptoimidazole

Reaction of 3,5-difluorobenzaldehyde (14.7 g, 0.104 mole) and aminoacetaldehyde dimethyl acetal (10.8 g, 0.104 mole) substantially as above yielded 10.0 g (43%) of the title compound as white crystals: m.p. 140-1° (ethyl acetate/hexane).

Example 23

1-[3-(3′,5′-Difluorophenyl)propyl]-2-mercaptoimidazole

3,5-Difluorobenzaldehyde (5.5 g, 0.039 mole), malonic acid (6.06 g, 0.058 mole), pyridine (2.1 ml) and piperidine (0.105 ml) were heated on a steam bath for 2 hours and then at 155° for 1 hour. The reaction mixture was poured into cold 3N aqueous hydrochloric acid, and then filtered. Recrystallization from ethanol provided 4.7 g (66%) of 3,5-difluorocinnamic acid as buff needles: m.p. 199-201°. 3-5-Difluorocinnamic acid (4.6 g, 0.025 mole) was dissolved in tetrahydrofuran (50 ml) and added to a slurry of 0.75 g palladium/carbon in ethyl acetate. The mixture was shaken under 50 psi (.34 MPa) hydrogen for 5 hours and then was filtered and concentrated to provide 4.5 g (97%) of 3-[3′,5′-difluorophenyl] propanoic acid as colorless crystals: m.p. 56° (methanol).

A solution of 3-[3′5′-difluorophenyl]propanoic acid (4.4 g, 0.024 mole) N,N-dimethylformamide (one drop) and thionyl chloride (15 ml) was heated at 60°. for 3 hours. Excess thionyl chloride was removed by distillation at reduced pressure. Distillation (Kugelrohr) at reduced Pressure (about 0.25 mm (33 Pa)) yielded 4.1 g (85%) of 3-[3′,5′-difluorophenyl]propionyl chloride as an oil.

A solution of 3-[3′,5′-difluorophenyl]proprionyl chloride (4 g, 0.0196 mole) in methylene chloride (40 ml) was slowly added to a 0° solution of amino acetaldehyde dimethylacetal (4.3 g, 0.0412 mole) in methylene chloride (100 ml) at a rate such that the temperature did not exceed 20°. The reaction mixture was stirred for 1 hour. Then it was poured into water and the layers were separated. The organic layer was washed with 5% aqueous sodium carbonate, 0.05% aqueous hydrogen chloride, and water and then was dried over sodium sulfate and concentrated to yield 5.5 g (103%) of 3-[3′,5′-difluorophenyl]propanamide N-acetaldehyde dimethyl acetal as an oil.

A solution of 3-[3′,5′-difluorophenyl]propanamide N-acetaldehyde dimethyl acetal (5.3 g, 0.0194 mole) in diethyl ether (100 ml) was slowly added to a slurry of lithium aluminum hydride (4.4 g, 0.116 mole) in diethyl ether (200 ml). The reaction mixture was stirred at ambient temperature (20-25°) for 18 hours. Then water (4.5 ml, was carefully added, followed by 10% aqueous sodium hydroxide (7 ml) and water (11 ml). The mixture was filtered and the filtrate was dried over sodium sulfate and concentrated to yield 4.4 g (88%) of 3-[3′,5′-difluorophenyl]propanamine N-acetaldehyde dimethylacetal as colorless oil.

A solution of 3-[3′,5′-difluorophenyl]propanamine N-acetaldehyde dimethyl acetal (4.3 g, 0.0166 mole)

and potassium thiocyanate (1.6 g, 0.0166 mol) in ethanol (12 ml), water (20 ml), and concentrated hydrochloric acid (4 ml) was refluxed for 1 hour and then cooled and a large volume of water was added. The product was filtered and recrystallized to yield 2.2 g (55%) of 1-[3-(3',5'-difluorophenyl)propyl]-2-mercaptoimidazole as white needles: m.p. 131-132° (ethanol).

Example 24

3-(3',5'-Dichlorophenyl)propyl-2-mercaptoimidazole

Reaction of 3,5-dichlorobenzaldehyde (26.9 g, 0.154 mole), malonic acid (24.1 g, 0.232 mole), pyridine (8 ml) and piperidine (0.4 ml) substantially as above yielded 22.9 g (69%) of 3,5-dichlorocinnamic acid as white needles: m.p. 169-170° (ethanol).

Reaction of 3,5-dichlorocinnamic acid (22.9 g, 0.106 mol) and 3 g palladium/carbon substantially as above yielded 23 g (99%) of 3-(3',5'-dichlorophenyl)propanoic acid as an oil.

A one molar solution of borane in tetrahydrofuran (233 ml) was added dropwise to a cooled (0°) solution of 3-(3',5'-dichlorophenyl)propanoic acid (23 g, 0.106 mole) in distilled tetrahydrofuran (200 ml). The reaction was stirred at room temperature for 2 hours. Then methanol was added and the solution was concentrated to yield 21.2 g (98%) of 1-[3-(3',5'-dichlorophenyl)]propanol as a clear oil.

Dimethyl sulfoxide (6.75 g, 0.083 mole) in dry methylene chloride (15 ml) was added dropwise to a solution of oxalylchloride (6.2 g, 0.049 mole) in dry methylene chloride (15 ml) at -78°. The reaction mixture was stirred for 2 minutes. Then 1-[3-(3',5'-dichlorophenyl)]-propanol (5 g, 0.0245 mole) in dry methylene chloride (20 ml) was slowly added, keeping the temperature below -60°. After stirring for 15 minutes at -70°, triethylamine (16 g, 0.160 mol) was added dropwise. The reaction mixture was stirred for an additional 5 minutes at -60° and then was warmed to room temperature and diluted with water. The organic layer was separated, washed with 3N aqueous hydrogen chloride and then with brine, and was dried over sodium sulfate. The solution was concentrated to give 5.0 g (100%) of 3-(3',5'-dichlorophenyl)propionaldehyde as a yellow oil.

Amino acetaldehyde dimethylacetal (2.1 g, 0.0197 mole) was added with stirring to a solution of 3-(3',5'-dichlorophenyl)propionaldehyde (5 g, 0.025 mole) in hexane (10 ml). After stirring for 1 hour at room temperature, sodium borohydride (7.3 g, 0.193 mole) in ethanol (25 ml) was added. The reaction mixture was stirred for 18 hours and then was diluted with water and concentrated. The residue was taken up in ethylacetate, washed with water, dried over sodium sulfate and concentrated to yield 6.8 g (93%) of 3-(3',5'-dichlorophenyl)propanamide N-acetaldehyde dimethyl acetal as a yellow oil.

A solution of 3-(3',5'-dichlorophenyl)propanamide N-acetaldehyde dimethyl acetal and potassium thiocynate (2.2 g, 0.0223 mole) in ethanol (20 ml), water (30 ml), and concentrated hydrochloric acid was refluxed for 1 hour. The reaction mixture was cooled and diluted with water. After standing for 3 hours, the crude product solidified and was filtered. Chromatography on silica, eluting with 0.5 to 1% methanol in methylene chloride, provided 2.0 g (31%) of 3-(3',5'-dichlorophenyl)propyl2-mercaptoimidazole as white crystals: m.p. 98-99° (ethanol).

While the preferred embodiments of the invention are illustrated by the above, it is to be understood that the invention is not limited to the precise constructions herein disclosed and that the right to all modifications coming within the scope of the following claims is reserved.

## Claims

1. A compound of formula (III):

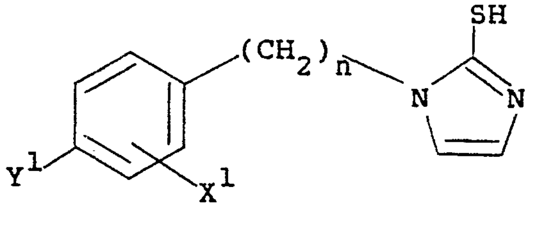

(III)

in which:
$Y^1$ is $-OCH_3$;
$X^1$ is -H, halogen, $C_{1-4}$ alkyl, -CN, $-NO_2$, $-SO_2NH_2$, $-CO_2H$, $-CONH_2$, -CHO, $-CH_2OH$, $-CF_3$, $-OCH_3$, $-SO_2C_{1-4}$ alkyl, $-SO_2C_{1-4}$ fluoralkyl, or $-CO_2C_{1-4}$ alkyl; or any synthetically accessible combination thereof up to four substituents; and
n is 0-4.

2. A compound according to Claim 1 in which n is 1 or 3, $X^1$ is hydrogen, methoxy or halogen.

3. A compound according to Claim 2 in which $X^1$ is hydrogen, methoxy, 3,5-dichloro-,3,5-difluoro, 3-chloro or 3-fluoro.

4. A compound according to Claim 3 in which $X^1$ is 3-5-difluoro and n is 1.

Claims for the following Contracting State :AT:

1. A process for the preparation of a compound of formula III:

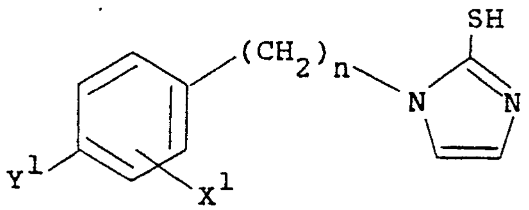

(III)

in which:
$Y^1$ is $-OCH_3$;
$X^1$ is -H, halogen, $C_{1-4}$ alkyl, -CN, $-NO_2$, $-SO_2NH_2$, $-CO_2H$, $-CONH_2$, -CHO, $-CH_2OH$, $-CF_3$, $-OCH_3$, $-SO_2C_{1-4}$ alkyl, $-SO_2C_{1-4}$ fluoralkyl, or $-CO_2C_{1-4}$ alkyl; or any synthetically accessible combination thereof up to four substituents; and
n is 0-4.
which comprises cyclisation of a compound of structure (II):

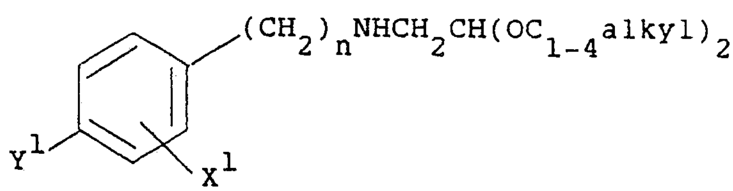

(II)

in which $X^1$, $Y^1$ and n are as described for structure (III).

2. A process according to Claim 1 in which n is 1 or 3, $X^1$ is hydrogen, methoxy or halogen.

3. A process according to Claim 2 in which $X^1$ is hydrogen, methoxy, 3,5-dichloro-,3,5-difluoro, 3-chloro or 3-fluoro.

4. A process according to Claim 3 in which $X^1$ is 3-5-difluoro and n is 1.